# EUROPEAN PATENT SPECIFICATION

(11) **EP 3 298 016 B1**
(45) Date of publication and mention of the grant of the patent: **05.08.2020**
(21) Application number: 16796648.0
(22) Date of filing: 08.04.2016
(51) Int. Cl.: C07D 487/04, C07D 401/14, C07D 403/14, C07D 491/048, C07D 495/04, C09K 11/06, H01L 27/32, H01L 51/54, H05B 33/14, H01L 51/00, H05B 33/10, H01L 51/50

(54) **PHOSPHOROUS HOST MATERIAL AND ORGANIC ELECTROLUMINESCENT DEVICE COMPRISING THE SAME**
PHOSPHORHALTIGES WIRTSMATERIAL UND ORGANISCHE ELEKTROLUMINESZENTE VORRICHTUNG DAMIT
MATÉRIAU HÔTE PHOSPHORÉ ET DISPOSITIF ÉLECTROLUMINESCENT ORGANIQUE LE COMPRENANT

(30) Priority: 19.05.2015 KR 20150069699; 24.06.2015 KR 20150089958; 07.01.2016 KR 20160002155; 22.03.2016 KR 20160034173
(43) Date of publication of application: 28.03.2018
(73) Proprietor: Rohm And Haas Electronic Materials Korea Ltd., Cheonan-si, Chungcheongnam-do 31093 (KR)
(72) Inventor: LIM, Young-Mook, Korea 18449 (KR); LEE, Su-Hyun, Korea 18449 (KR); KANG, Hyun-Ju, Korea 18449 (KR); KIM, Chi-Sik, Korea 18449 (KR)
(74) Representative: Houghton, Mark Phillip
(86) International application number: PCT/KR2016/003716
(87) International publication number: WO 2016/186321

(56) References cited:
- EP-A1- 3 313 958
- WO-A1-2013/122402
- WO-A1-2015/046916
- WO-A1-2015/156587
- WO-A1-2015/156587
- WO-A1-2015/174738
- WO-A1-2015/174738
- WO-A1-2016/208873
- KR-A- 20150 110 101
- US-A1- 2012 068 170
- US-A1- 2014 374 711
- US-A1- 2015 060 796
- US-A1- 2015 318 510
- US-A1- 2015 318 510
- US-A1- 2016 005 979

## Description

### Technical Field

The present invention relates to phosphorous host materials and organic electroluminescent device comprising the same.

### Background Art

An electroluminescent device (EL device) is a self-light-emitting device which has advantages in that it provides a wider viewing angle, a greater contrast ratio, and a faster response time. The first organic EL device was developed by Eastman Kodak, by using small aromatic diamine molecules, and aluminum complexes as materials for forming a light-emitting layer [Appl. Phys. Lett. 51, 913, 1987].

The most important factor determining luminous efficiency in an organic EL device is light-emitting materials. Light-emitting materials are classified into fluorescent, phosphorous, and thermally activated delayed fluorescent (TADF) materials. In case of conventional carrier injection-type organic electroluminescent devices, electrons and holes injected from electrodes recombine at a light-emitting layer to form an exciton. Light is emitted as the exciton transfers to a ground state. Singlet state excitons and triplet state excitons are formed in a ratio of 1:3. A luminescence from the singlet state to the ground state is called fluorescence and a luminescence from the triplet state to the ground state is called phosphorous. It is difficult to observe phosphorous luminescence at room temperature in most of the organic compounds. However, when heavy element materials such as Ir, Pt, etc., are used, excitons in singlet states transfer to triplet states due to inter-system crossing (ISC), and all the excitons formed by recombination can be used in light-emission. Thus, 100% of internal quantum efficiency can be obtained.

Until now, fluorescent materials have been widely used as light-emitting material. However, in view of electroluminescent mechanisms, since phosphorescent materials theoretically enhance luminous efficiency by four (4) times compared to fluorescent materials, development of phosphorescent light-emitting materials is widely being researched. Iridium(III) complexes have been widely known as phosphorescent materials, including bis(2-(2'-benzothienyl)-pyridinato-N,C3')iridium(acetylacetonate) ((acac)Ir(btp)₂), tris(2-phenylpyridine)iridium (Ir(ppy)₃) and bis(4,6-difluorophenylpyridinato-N,C2)picolinate iridium (Firpic) as red, green, and blue materials, respectively.

At present, 4,4'-N,N'-dicarbazol-biphenyl (CBP) is the most widely known phosphorescent host material. Recently, Pioneer (Japan) et al. developed a high performance organic EL device using bathocuproine (BCP) and aluminum(III)bis(2-methyl-8-quinolinate)(4-phenylphenolate) (BAlq) etc., as host materials, which were known as hole blocking layer materials.

Although these materials provide good light-emitting characteristics, they have the following disadvantages: (1) Due to their low glass transition temperature and poor thermal stability, their degradation may occur during a high-temperature deposition process in a vacuum, and the lifespan of the device decreases. (2) The power efficiency of an organic EL device is given by [(π/voltage) × current efficiency], and the power efficiency is inversely proportional to the voltage. Although an organic EL device comprising phosphorescent host materials provides higher current efficiency (cd/A) than one comprising fluorescent materials, a significantly high driving voltage is necessary. Thus, there is no merit in terms of power efficiency (Im/W). (3) Further, the operational lifespan of an organic EL device is short and luminous efficiency is still required to be improved.

According to a recent study, through a design of molecules having very little excitation energy difference between singlet state and triplet state, thermally activated delayed fluorescence in which cross between a force system from a triplet state to a singlet state is possible using thermal energy.

A prior art of KR 1477613 B1 discloses a compound in which pyridine, pyrimidine, or triazine is bonded to a nitrogen atom of carbazole fused with indole, directly or via a linker of phenylene. However, said reference does not specifically disclose a compound in which pyridine, pyrimidine, or triazine is bonded to a nitrogen atom of carbazole fused with indole, via a linker of pyridylene or pyrimidinylene.

A prior art of KR 1317923 B1 discloses a compound in which pyridine is bonded to a nitrogen atom of carbazole fused with indole, via a linker of pyrimidinylene. However, said reference does not disclose any example using the compound as a phosphorous host material.

US2015/318510 discloses an organic light-emitting device includes an anode, a cathode, and an organic layer between the anode and the cathode, wherein the organic layer includes a mixed organic layer, and the mixed organic layer includes at least two different compounds, and a triplet energy of at least one compound of the at least two different compounds is 2.2 eV or higher.

### Disclosure of the Invention

### Problems to be Solved

The objective of the present invention is first, to provide a phosphorous host material capable of producing an organic electroluminescent device with excellent lifespan characteristics, and second, to provide an organic electroluminescent device comprising the phosphorous host material.

### Solution to Problems

The invention is set out in accordance with the appended claims. The present inventors found that the above objective can be achieved by a phosphorous host material comprising a compound represented by the following formula 1: 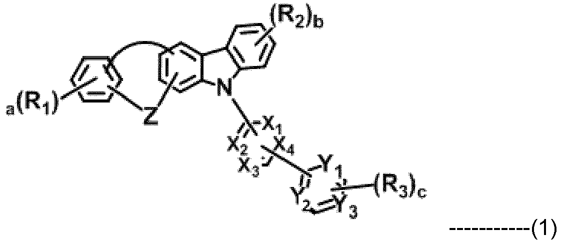 wherein
Z represents O, or S;
X₁ to X₄ each independently represent N or C(R₇), one or more of which are N;
Y₁ to Y₃ each independently represent N or C(R₈), two or more of which are N;
R₁ to R₄ and R₈ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen, and sulfur;
R₇ represents hydrogen;
a and b each independently represent an integer of 1 to 4;
c represents 1 or 2;
where a, b, or c is an integer of 2 or more, each of R₁, each of R₂, or each of R₃ may be the same or different; and
the heteroaryl contains at least one hetero atom selected from B, N, O, S, Si, and P.

### Effects of the Invention

By using the phosphorous host material of the present invention, an organic electroluminescent device having significantly improved operational lifespan can be produced.

### Embodiments of the Invention

Hereinafter, the present invention will be described in detail. However, the following description is intended to explain the invention, and is not meant in any way to restrict the scope of the invention.

The present invention relates to a phosphorous host material comprising a compound represented by formula 1, and an organic electroluminescent device comprising the material.

Hereinafter, the compound represented by formula 1 will be described in detail.

The compound represented by formula 1 can be represented by one of the following formulas 2 to 6: 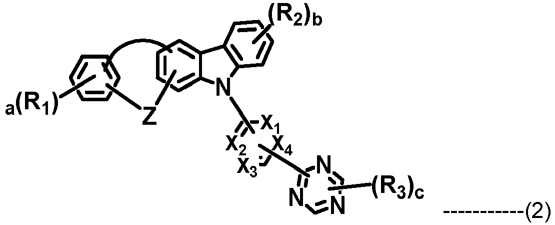 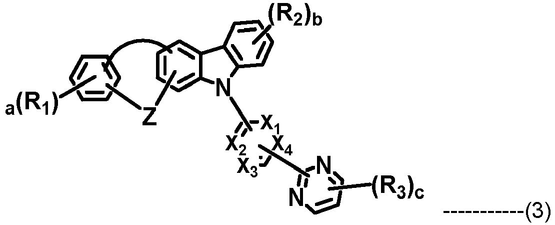 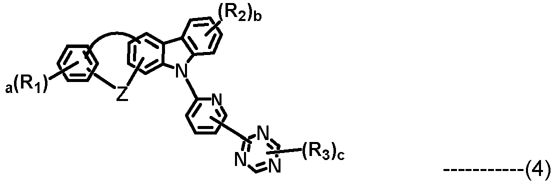 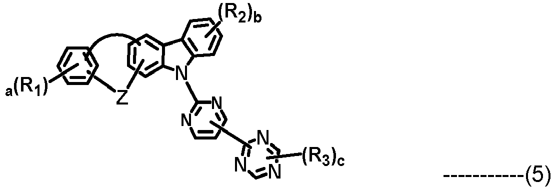 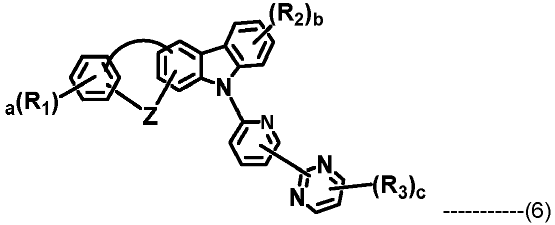 wherein
R₁ to R₃, X₁ to X₄, Z, and a to c are as defined in formula 1.

The structure of 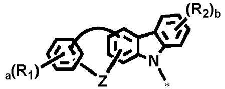 in formula 1 can be represented by one of the following formulas 7 to 12: 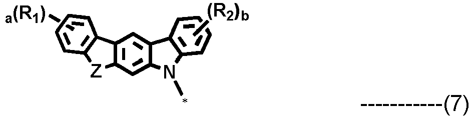 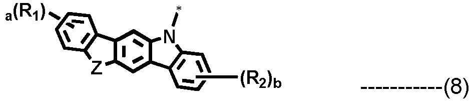 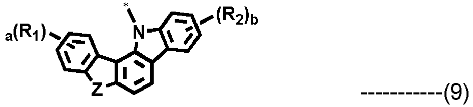 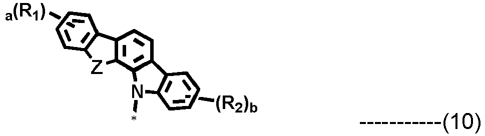 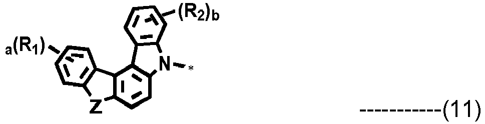 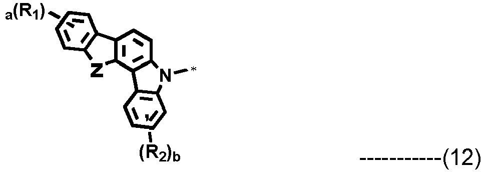 wherein
R₁, R₂, Z, a, and b are as defined in formula 1.

Herein, "(C1-C30)alkyl" is meant to be a linear or branched alkyl having 1 to 30 carbon atoms constituting the chain, in which the number of carbon atoms is preferably 1 to 10, more preferably 1 to 6, and includes methyl, ethyl, n-propyl, isopropyl, n-butyl, isobutyl, tert-butyl, etc.; "(C2-C30)alkenyl" is meant to be a linear or branched alkenyl having 2 to 30 carbon atoms constituting the chain, in which the number of carbon atoms is preferably 2 to 20, more preferably 2 to 10, and includes vinyl, 1-propenyl, 2-propenyl, 1-butenyl, 2-butenyl, 3-butenyl, 2-methylbut-2-enyl, etc.; "(C2-C30)alkynyl" is meant to be a linear or branched alkynyl having 2 to 30 carbon atoms constituting the chain, in which the number of carbon atoms is preferably 2 to 20, more preferably 2 to 10, and includes ethynyl, 1-propynyl, 2-propynyl, 1-butynyl, 2-butynyl, 3-butynyl, 1-methylpent-2-ynyl, etc.; "(C3-C30)cycloalkyl" is a mono- or polycyclic hydrocarbon having 3 to 30 ring backbone carbon atoms, in which the number of carbon atoms is preferably 3 to 20, more preferably 3 to 7, and includes cyclopropyl, cyclobutyl, cyclopentyl, cyclohexyl, etc.; "3- to 7- membered heterocycloalkyl" is a cycloalkyl having 3 to 7 ring backbone atoms, including at least one heteroatom selected from B, N, O, S, Si, and P, preferably O, S, and N, and includes tetrahydrofuran, pyrrolidine, thiolan, tetrahydropyran, etc.; "(C6-C30)aryl(ene)" is a monocyclic or fused ring derived from an aromatic hydrocarbon having 6 to 30 ring backbone carbon atoms, in which the number of carbon atoms is preferably 6 to 20, more preferably 6 to 15, and includes phenyl, biphenyl, terphenyl, naphthyl, binaphthyl, phenylnaphthyl, naphthylphenyl, phenylterphenyl, fluorenyl, phenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, etc.; "3- to 30-membered heteroaryl(ene)" is an aryl having 3 to 30 ring backbone atoms, including at least one, preferably 1 to 4 heteroatoms selected from the group consisting of B, N, O, S, Si, and P; is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., and a fused ring-type heteroaryl including benzofuranyl, benzothiophenyl, isobenzofuranyl, dibenzofuranyl, dibenzothiophenyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenoxazinyl, phenanthridinyl, benzodioxolyl, etc. Further, "halogen" includes F, Cl, Br, and I.

Herein, "substituted" in the expression, "substituted or unsubstituted," means that a hydrogen atom in a certain functional group is replaced with another atom or group, i.e. a substituent. In the present invention, the substituents of the substituted (C1-C30)alkyl, the substituted (C6-C30)aryl, the substituted 3- to 30-membered heteroaryl, the substituted (C3-C30)cycloalkyl, the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di- (C6-C30)arylamino, the substituted (C1-C30)alkyl(C6-C30)arylamino, and the substituted mono- or polycyclic, (C3-C30) alicyclic or aromatic ring in R₁ to R₈ in formula 1 each independently are at least one selected from the group consisting of deuterium, a halogen, a cyano, a carboxyl, a nitro, a hydroxyl, a (C1-C30)alkyl, a halo(C1-C30)alkyl, a (C2-C30) alkenyl, a (C2-C30) alkynyl, a (C1-C30)alkoxy, a (C1-C30)alkylthio, a (C3-C30)cycloalkyl, a (C3-C30)cycloalkenyl, a 3- to 7-membered heterocycloalkyl, a (C6-C30)aryloxy, a (C6-C30)arylthio, a 5- to 30-membered heteroaryl unsubstituted or substituted with a (C6-C30)aryl, a (C6-C30)aryl unsubstituted or substituted with a 5- to 30-membered heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di-(C1-C30)alkylamino, a mono- or di-(C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

Z represents O, or S.

X₁ to X₄ each independently represent N or C(R₇), one or more of which are N.

Y₁ to Y₃ each independently represent N or C(R₈), two or more of which are N.

R₁ to R₄ and R₈ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen, and sulfur; preferably each independently represent hydrogen, a substituted or unsubstituted (C1-C6)alkyl, or a substituted or unsubstituted (C6-C15)aryl; or are linked to an adjacent substituent(s) to form a substituted or unsubstituted, mono- or polycyclic, (C3-C12) aromatic ring; and more preferably R₁ and R₂ each independently represent hydrogen; or are linked to an adjacent substituent(s) to form a substituted or unsubstituted, monocyclic (C3-C12) aromatic ring, R₃ to R₆ each independently represent an unsubstituted (C1-C6)alkyl, or a (C6-C15)aryl unsubstituted or substituted with a (C1-C6)alkyl, and R₇ and R₈ each independently represent hydrogen.

According to one embodiment of the present invention, in formula 1 above, Z represents O, or S; X₁ to X₄ each independently represent N or C(R₇), one or more of which are N; Y₁ to Y₃ each independently represent N or C(R₈), two or more of which are N; and R₁ to R₈ each independently represent hydrogen, a substituted or unsubstituted (C1-C6)alkyl, or a substituted or unsubstituted (C6-C15)aryl; or are linked to an adjacent substituent(s) to form a substituted or unsubstituted, mono- or polycyclic, (C3-C12) aromatic ring.

According to another embodiment of the present invention, in formula 1 above, Z represents O, or S; X₁ to X₄ each independently represent N or C(R₇), one or more of which are N; Y₁ to Y₃ each independently represent N or C(R₈), two or more of which are N; R₁ and R₂ each independently represent hydrogen; or are linked to an adjacent substituent(s) to form a substituted or unsubstituted, monocyclic (C3-C12) aromatic ring; R₃ to R₆ each independently represent an unsubstituted (C1-C6)alkyl, or a (C6-C15)aryl unsubstituted or substituted with a (C1-C6)alkyl; and R₇ and R₈ each independently represent hydrogen.

The compound represented by formula 1 includes the following compounds, but is not limited thereto:

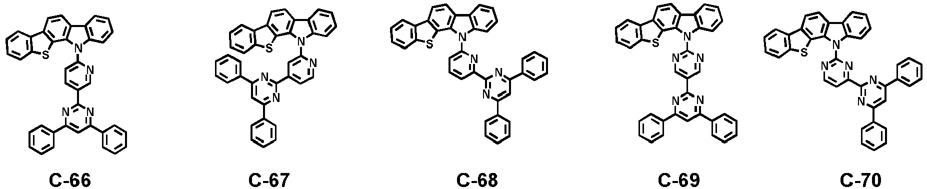

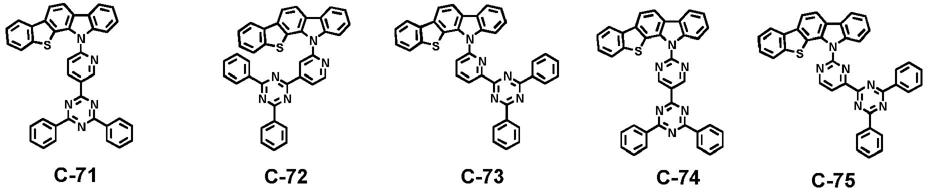

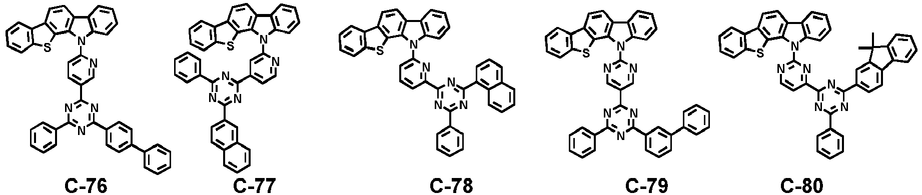

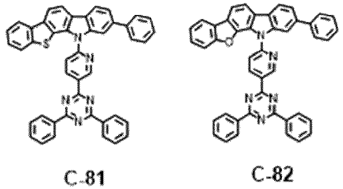

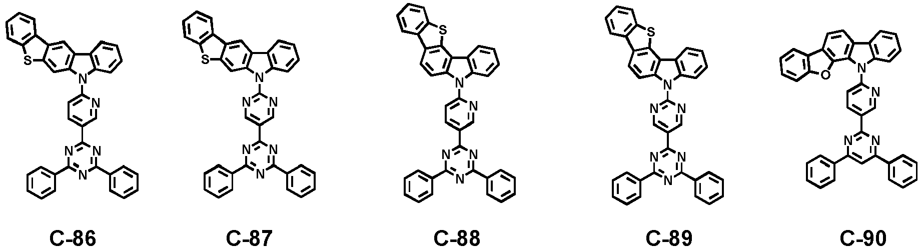

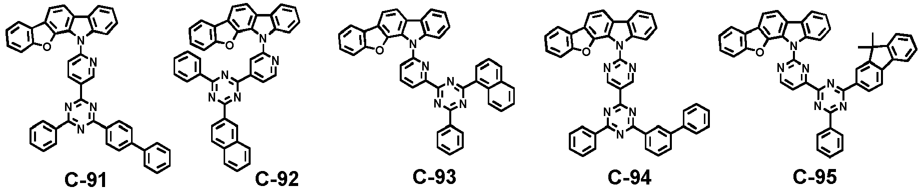

The compound of formula 1 according to the present invention can be prepared by a synthetic method known to a person skilled in the art. For example, it can be prepared according to the following reaction scheme. 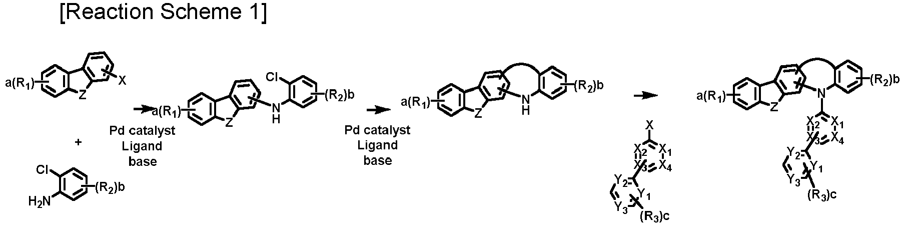 wherein R₁ to R₃, Z, X₁ to X₄, Y₁ to Y₃, and a to c are as defined in formula 1, and X represents halogen.

The present invention provides a phosphorous host material comprising the compound of formula 1, and an organic electroluminescent device comprising the material.

The above material can be comprised of the compound of formula 1 alone, or can further include conventional materials generally used in phosphorous host materials.

The organic electroluminescent device comprises a first electrode; a second electrode; and at least one organic layer between the first and second electrodes. The organic layer may comprise the phosphorous host material of the present invention.

## Claims

1. A phosphorous host material comprising a compound represented by the following formula 1: 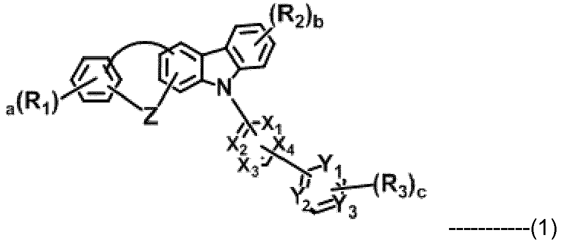 wherein
Z represents O, or S;
X₁ to X₄ each independently represent N or C(R₇), one or more of which are N;
Y₁ to Y₃ each independently represent N or C(R₈), two or more of which are N;
R₁ to R₄ and R₈ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent(s) to form a substituted or unsubstituted, mono- or polycyclic, (C3-C30) alicyclic or aromatic ring, whose carbon atom(s) may be replaced with at least one hetero atom selected from nitrogen, oxygen, and sulfur;
R₇ represents hydrogen;
a and b each independently represent an integer of 1 to 4;
c represents 1 or 2;
where a, b, or c is an integer of 2 or more, each of R₁, each of +R₂, or each of R₃ may be the same or different; and
the heteroaryl contains at least one hetero atom selected from B, N, O, S, Si, and P.

2. The phosphorous host material according to claim 1, wherein the compound represented by formula 1 is represented by one of the following formulas 2 to 6: 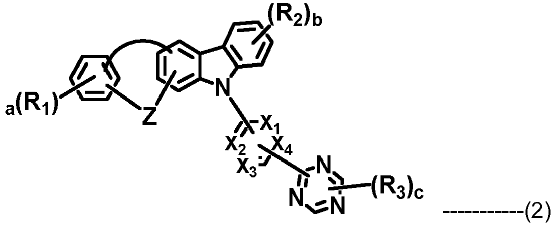 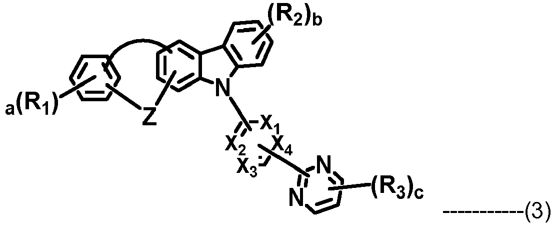 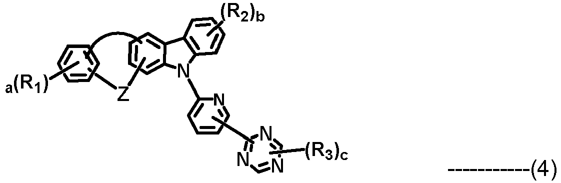 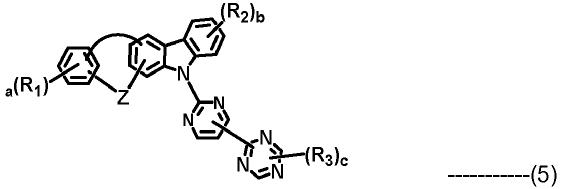 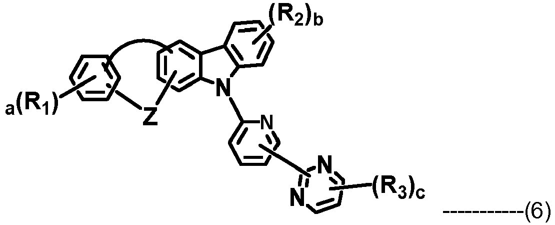 wherein
R₁ to R₃, X₁ to X₄, Z, and a to c are as defined in claim 1.

3. The phosphorous host material according to claim 1, wherein the structure of 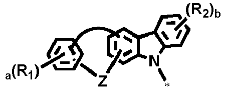 in formula 1 is represented by one of the following formulas 7 to 12: 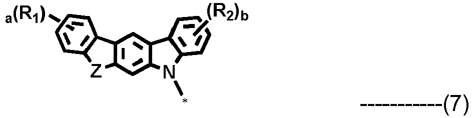 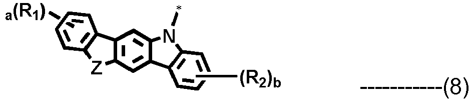 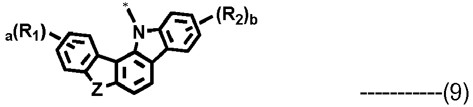 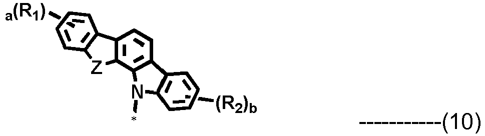 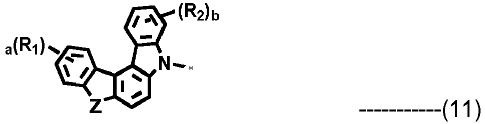 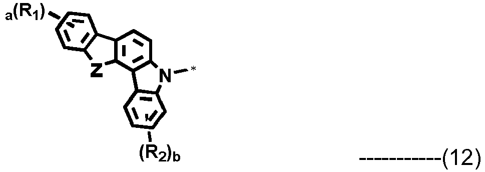 wherein
R₁, R₂, Z, a, and b are as defined in claim 1.

4. The phosphorous host material according to claim 1, wherein the substituents of the substituted (C1-C30)alkyl, the substituted (C6-C30)aryl, the substituted 3- to 30-membered heteroaryl, the substituted (C3-C30)cycloalkyl, the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di- (C6-C30)arylamino, the substituted (C1-C30)alkyl(C6-C30)arylamino, and the substituted mono- or polycyclic, (C3-C30) alicyclic or aromatic ring in R₁ to R₄ and R₈ each independently are at least one selected from the group consisting of deuterium, a halogen, a cyano, a carboxyl, a nitro, a hydroxyl, a (C1-C30)alkyl, a halo(C1-C30)alkyl, a (C2-C30) alkenyl, a (C2-C30) alkynyl, a (C1-C30)alkoxy, a (C1-C30)alkylthio, a (C3-C30)cycloalkyl, a (C3-C30)cycloalkenyl, a 3- to 7-membered heterocycloalkyl, a (C6-C30)aryloxy, a (C6-C30)arylthio, a 5- to 30-membered heteroaryl unsubstituted or substituted with a (C6-C30)aryl, a (C6-C30)aryl unsubstituted or substituted with a 5- to 30-membered heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di- (C1-C30)alkylamino, a mono- or di-(C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

5. The phosphorous host material according to claim 1, wherein;
R₁ to R₄ and R₈ each independently represent hydrogen, a substituted or unsubstituted (C1-C6)alkyl, or a substituted or unsubstituted (C6-C15)aryl; or are linked to an adjacent substituent(s) to form a substituted or unsubstituted, mono- or polycyclic, (C3-C12) aromatic ring.

6. The phosphorous host material according to claim 1, wherein
R₃ to R₄ each independently represent an unsubstituted (C1-C6)alkyl, or a (C6-C15)aryl unsubstituted or substituted with a (C1-C6)alkyl; and
R₇ and R₈ each independently represent hydrogen.

7. The phosphorous host material according to claim 1, wherein the compound represented by formula 1 is selected from the group consisting of:

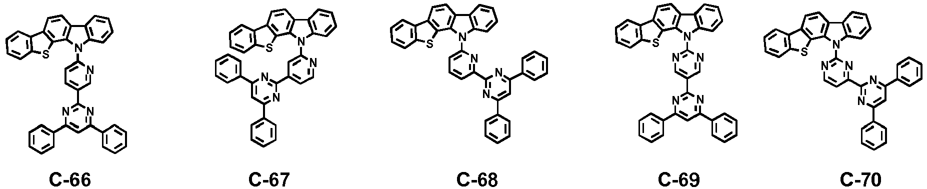

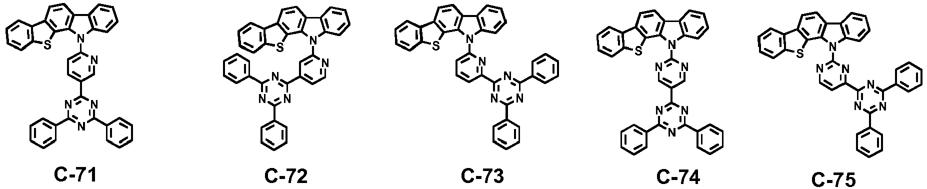

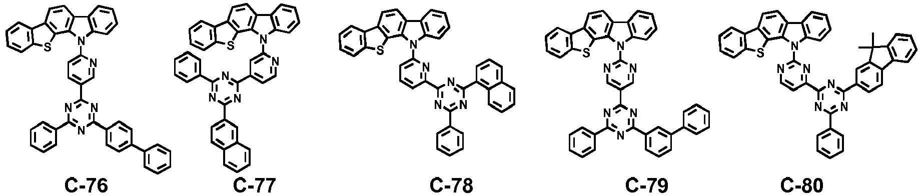

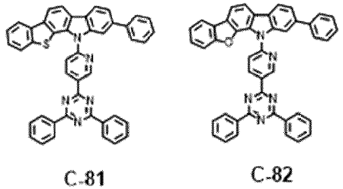

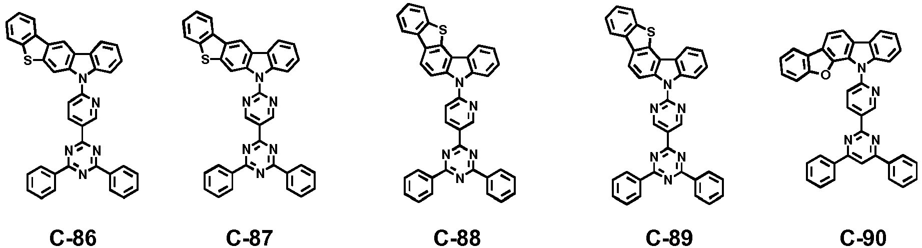

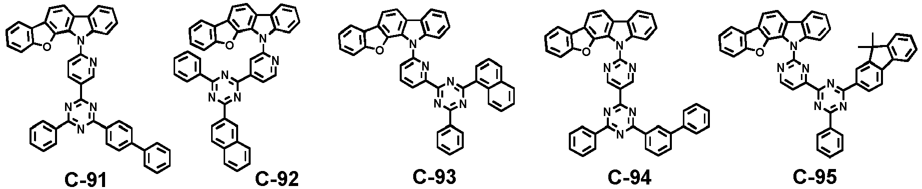

8. An organic electroluminescent device comprising the phosphorous host material according to claim 1.

## Patentansprüche

1. Phosphor enthaltendes Wirtsmaterial, umfassend eine Verbindung, die durch die folgende Formel 1 dargestellt wird: 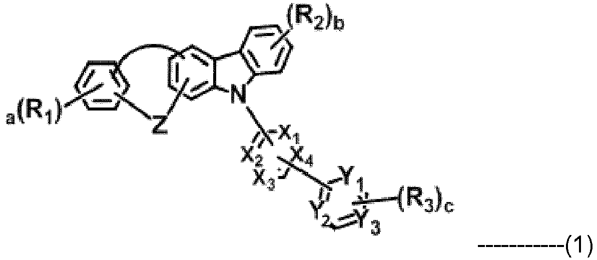 wobei:
Z O oder S darstellt;
X₁ bis X₄ jeweils unabhängig N oder C(R₇) darstellen, von denen ein oder mehrere N sind;
Y₁ bis Y₃ jeweils unabhängig N oder C(R₈) darstellen, von denen zwei oder mehrere N sind;
R₁ bis R₄ und R₈ jeweils unabhängig Wasserstoff, Deuterium, ein Halogen, ein Cyano, ein substituiertes oder unsubstituiertes (C1-C30)Alkyl, ein substituiertes oder unsubstituiertes (C6-C30)Aryl, ein substituiertes oder unsubstituiertes 3- bis 30-gliedriges Heteroaryl, ein substituiertes oder unsubstituiertes (C3-C30)Cycloalkyl, ein substituiertes oder unsubstituiertes (C1-C30)Alkoxy, ein substituiertes oder unsubstituiertes Tri(C1-C30)alkylsilyl, ein substituiertes oder unsubstituiertes Di(Cl-C30)alkyl(C6-C30)arylsilyl, ein substituiertes oder unsubstituiertes (Cl-C30)Alkyldi(C6-C30)arylsilyl, ein substituiertes oder unsubstituiertes Tri(C6-C30)arylsilyl, ein substituiertes oder unsubstituiertes Mono- oder Di(Cl-C30)alkylamino, ein substituiertes oder unsubstituiertes Mono- oder Di(C6-C30)arylamino oder ein substituiertes oder unsubstituiertes (Cl-C30)Alkyl(C6-C30)arylamino darstellen; oder mit einem (mehreren) angrenzenden Substituenten verknüpft sind, um einen substituierten oder unsubstituierten, mono- oder polycyclischen, (C3-C30)-alicyclischen oder aromatischen Ring zu bilden, dessen Kohlenstoffatom(e) durch mindestens ein Heteroatom ersetzt werden können, das aus Stickstoff, Sauerstoff und Schwefel ausgewählt ist;
R₇ Wasserstoff darstellt;
a und b jeweils unabhängig eine ganze Zahl von 1 bis 4 darstellen;
c 1 oder 2 darstellt;
worin, wenn a, b oder c eine ganze Zahl von 2 oder mehr ist, jedes R₁, jedes R₂ oder jedes R₃ gleich oder verschieden sein kann; und
das Heteroaryl mindestens ein Heteroatom enthält, das ausgewählt ist aus B, N, O, S, Si und P.

2. Phosphor enthaltendes Wirtsmaterial nach Anspruch 1, wobei die durch Formel 1 dargestellte Verbindung durch eine der Formeln 2 bis 6 dargestellt wird: 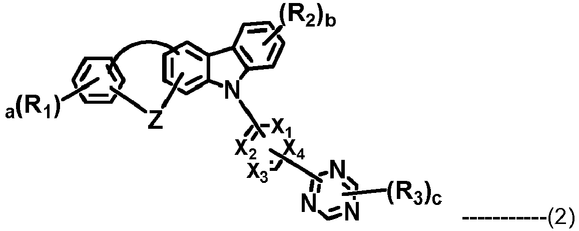 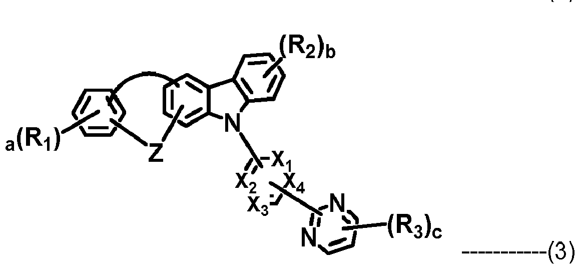 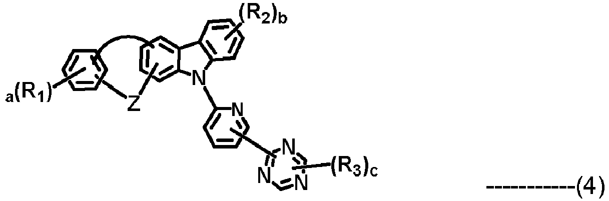 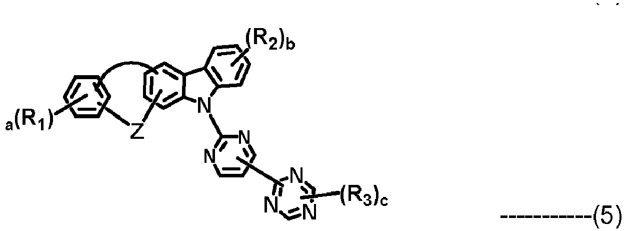 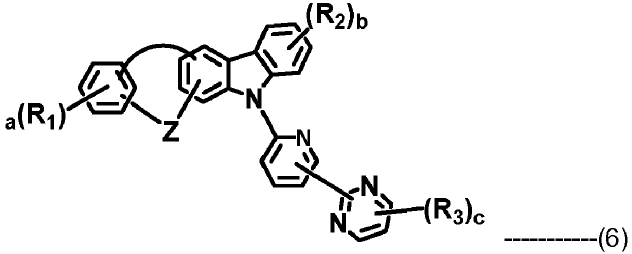 wobei
R₁ bis R₃, X₁ bis X₄, Z und a bis c wie in Anspruch 1 festgelegt sind.

3. Phosphor enthaltendes Wirtsmaterial nach Anspruch 1, wobei die Struktur von 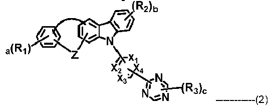 in Formel 1 durch eine der folgenden Formeln 7 bis 12 dargestellt wird: 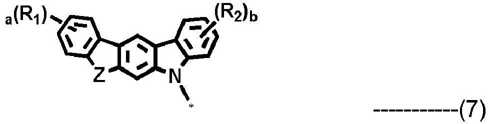 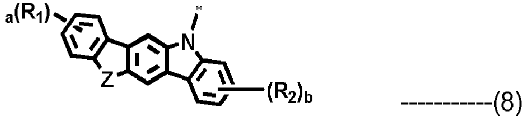 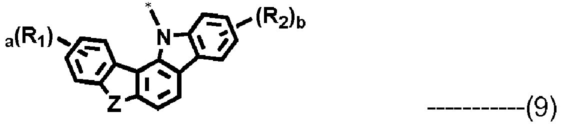 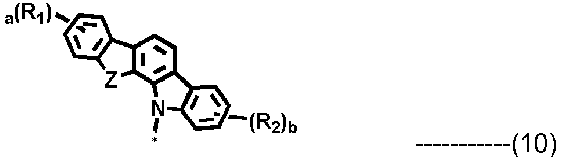 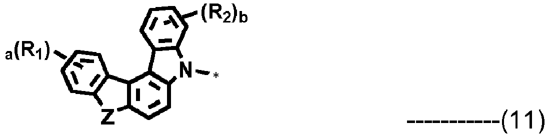 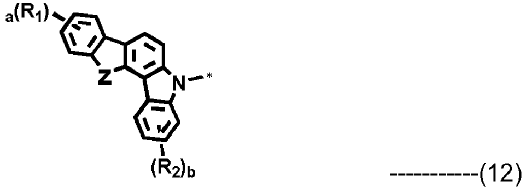 wobei
R₁, R₂, Z, a und b wie in Anspruch 1 festgelegt sind.

4. Phosphor enthaltendes Wirtsmaterial nach Anspruch 1, wobei die Substituenten des substituierten (C1-C30)Alkyls, des substituierten (C6-C30)Aryls, des substituierten 3-bis 30-gliedrigen Heteroaryls, des substituierten (C3-C30)Cycloalkyls, des substituierten (C1-C30)Alkoxys, des substituierten Tri(C1-C30)alkylsilyls, des substituierten Di(C1-C30)alkyl(C6-C30)arylsilyls, des substituierten (C1-C30)Alkyldi(C6-C30)arylsilyls, des substituierten Tri(C6-C30)arylsilyls, des substituiertem Mono- oder Di(C1-C30)alkylaminos, des substituierten Mono- oder Di(C6-C30)arylaminos, des substituierten (C1-C30)Alkyl(C6-C30)arylaminos und des substituierten, mono- oder polycyclischen, (C3-C30)-alicyclischen oder aromatischen Rings in R₁ bis R₄ und R₈ jeweils unabhängig mindestens eine aus der Gruppe bestehend aus Deuterium, einem Halogen, einem Cyano, einem Carboxyl, einem Nitro, einem Hydroxyl, einem (C1-C30)Alkyl, einem (C1-C30)Halogenalkyl, einem (C2-C30)Alkenyl, einem (C2-C30)Alkinyl, einem (C1-C30)Alkoxy, einem (C1-C30)Alkylthio, einem (C3-C30)Cycloalkyl, einem (C3-C30)Cycloalkenyl, einem 3- bis 7-gliedrigen Heterocycloalkyl, einem (C6-C30)Aryloxy, einem (C6-C30)Arylthio, einem 5- bis 30-gliedrigen Heteroaryl, das unsubstituiert ist oder mit einem (C6-C30)Aryl substituiert ist, einem (C6-C30)Aryl, das unsubstituiert ist oder mit einem 5- bis 30-gliedrigen Heteroaryl substituiert ist, einem Tri(C1-C30)alkylsilyl, einem Tri(C6-C30)arylsilyl, einem Di(C1-C30)alkyl(C6-C30)arylsilyl, einem (C1-C30)Alkyldi(C6-C30)arylsilyl, einem Amino, einem Mono- oder Di(C1-C30)alkylamino, einem Mono- oder Di(C6-C30)arylamino, einem (C1-C30)Alkyl(C6-C30)arylamino, einem (C1-C30)Alkylcarbonyl, einem (C1-C30)Alkoxycarbonyl, einem (C6-C30)Arylcarbonyl, einem Di(C6-C30)arylboronyl, einem Di(C1-C30)alkylboronyl, einem (C1-C30)Alkyl-(C6-C30)arylboronyl, einem (C6-C30)Aryl(C1-C30)alkyl und einem (C1-C30)Alkyl(C6-C30)aryl ausgewählt sind.

5. Phosphor enthaltendes Wirtsmaterial nach Anspruch 1, wobei:
R₁ bis R₄ und R₈ jeweils unabhängig Wasserstoff, ein substituiertes oder unsubstituiertes (C1-C6)Alkyl oder ein substituiertes oder unsubstituiertes (C6-C15)Aryl darstellen oder mit einem (mehreren) angrenzenden Substituenten verknüpft sind, um einen substituierten oder unsubstituierten, mono- oder polycyclischen, (C3-C12)-aromatischen Ring zu bilden.

6. Phosphor enthaltendes Wirtsmaterial nach Anspruch 1, wobei
R₃ bis R₄ jeweils unabhängig ein unsubstituiertes (C1-C6)Alkyl oder ein (C6-C15)Aryl darstellen, das unsubstituiert ist oder mit einem (C1-C6)Alkyl substituiert ist; und
R₇ und R₈ jeweils unabhängig Wasserstoff darstellen.

7. Phosphor enthaltendes Wirtsmaterial nach Anspruch 1, wobei die durch Formel 1 dargestellte Verbindung ausgewählt ist aus der Gruppe bestehend aus:

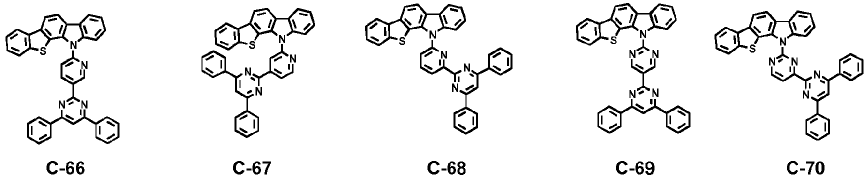

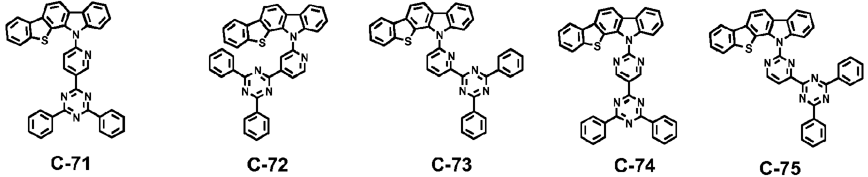

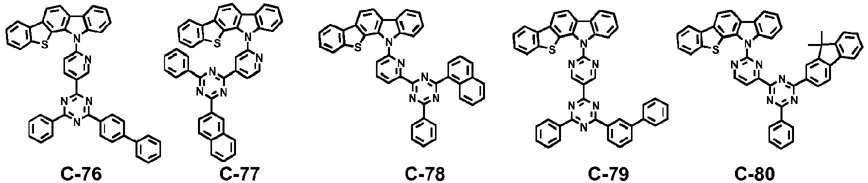

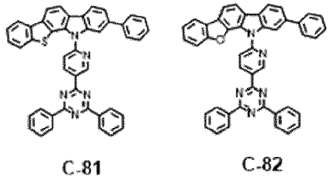

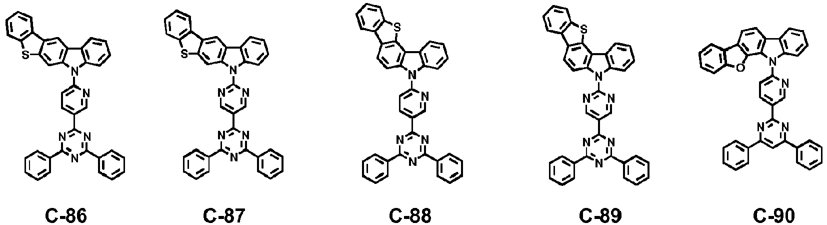

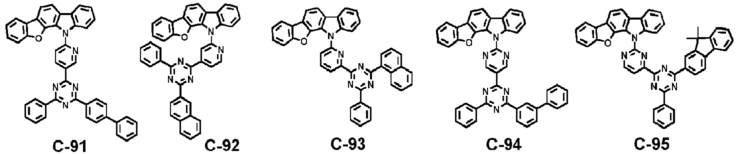

8. Organische elektrolumineszente Vorrichtung, umfassend das Phosphor enthaltende Wirtsmaterial nach Anspruch 1.

## Revendications

1. Matériau hôte phosphoré comprenant un composé représenté par la formule 1 suivante: 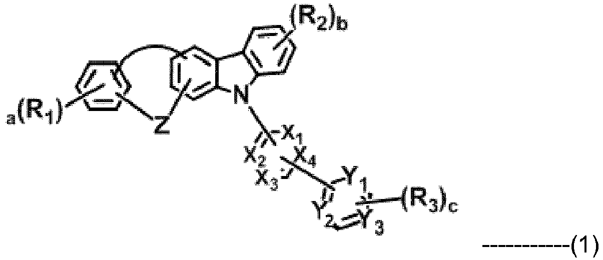 dans lequel
Z représente O ou S;
X₁ à X₄ représentent chacun indépendamment N ou C(R₇), dont un ou plus sont N;
Y₁ à Y₃ représentent chacun indépendamment N ou C(R₈), dont deux ou plus sont N;
R₁ à R₄ et R₈ représentent chacun indépendamment de l'hydrogène, du deutérium, un halogène, un cyano, un (C1-C30)alkyle substitué ou non substitué, un (C6-C30)aryle substitué ou non substitué, un hétéroaryle de 3 à 30 membres substitué ou non substitué, un (C3-C30)cycloalkyle substitué ou non substitué, un (C1-C30)alcoxy substitué ou non substitué, un tri(C1-C30)alkylsilyle substitué ou non substitué, un di(C1-C30)alkyl(C6-C30)arylsilyle substitué ou non substitué, un (C1-C30)alkyldi(C6-C30)arylsilyle substitué ou non substitué, un tri(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué; ou sont liés à un (ou plusieurs) substituant(s) adjacent(s) pour former un cycle (C3-C30) alicyclique ou aromatique, mono- ou polycyclique, substitué ou non substitué, dont le (ou les) atome(s) de carbone peu(ven)t être remplacé(s) avec au moins un hétéroatome choisi parmi azote, oxygène et soufre;
R₇ représente un hydrogène;
a et b représentent chacun indépendamment un nombre entier de 1 à 4;
c représente 1 ou 2;
lorsque a, b ou c est un nombre entier de 2 ou plus, chacun des R₁, chacun des R₂ ou chacun des R₃ peuvent être identiques ou différents; et
l'hétéroaryle contient au moins un hétéroatome choisi parmi B, N, O, S, Si et P.

2. Matériau hôte phosphoré selon la revendication 1, dans lequel le composé représenté par la formule 1 est représenté par l'une des formules 2 à 6 suivantes: 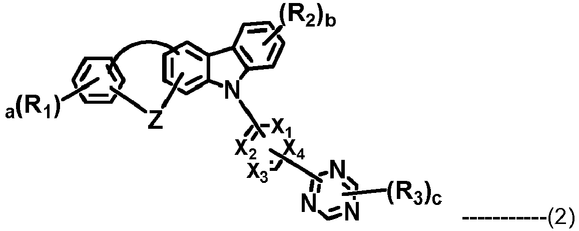 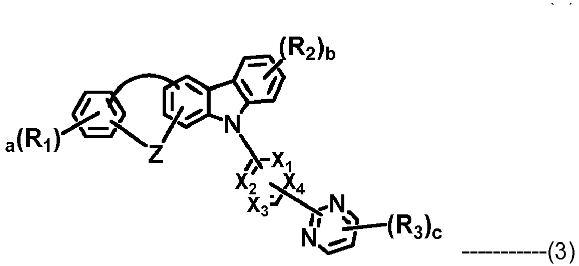 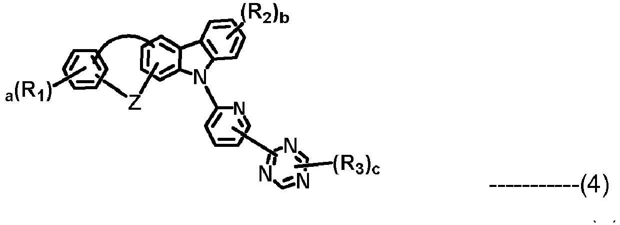 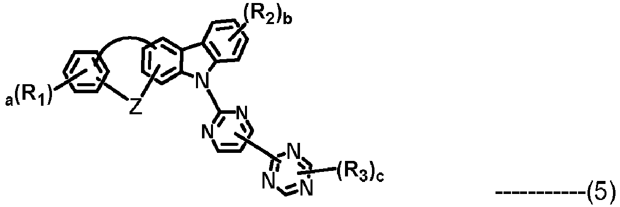 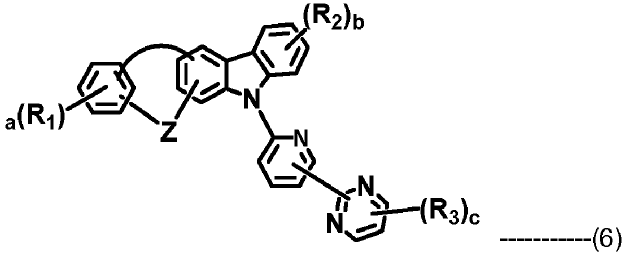 dans lequel
R₁ à R₃, X₁ à X₄, Z et a à c sont tels que définis dans la revendication 1.

3. Matériau hôte phosphoré selon la revendication 1, dans lequel la structure de 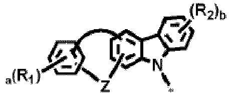 dans la formule 1 est représentée par l'une des formules 7 à 12 suivantes: 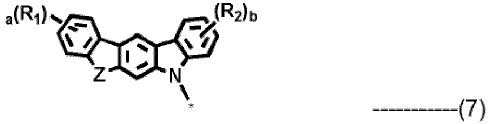 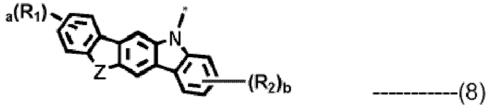 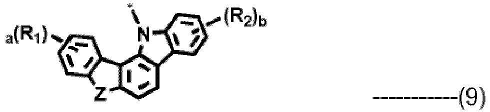 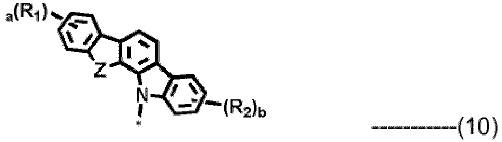 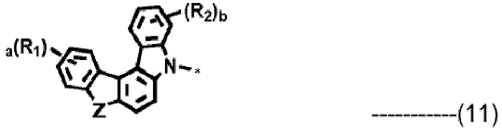 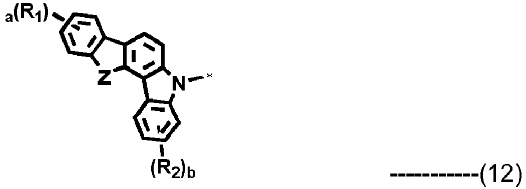 dans lequel
R₁, R₂, Z, a et b sont tels que définis dans la revendication 1.

4. Matériau hôte phosphoré selon la revendication 1, dans lequel les substituants du (C1-C30)alkyle substitué, du (C6-C30)aryle substitué, de l'hétéroaryle de 3 à 30 membres substitué, du (C3-C30)cycloalkyle substitué, du (C1-C30)alcoxy substitué, du tri(C1-C30)alkylsilyle substitué, du di(C1-C30)alkyl(C6-C30)arylsilyle substitué, du (C1-C30)alkyldi(C6-C30)arylsilyle substitué, du tri(C6-C30)arylsilyle substitué, du mono- ou di-(C1-C30)alkylamino substitué, du mono- ou di-(C6-C30)arylamino substitué, du (C1-C30)alkyl(C6-C30)arylamino substitué et du cycle (C3-C30) alicyclique ou aromatique, mono- ou polycyclique substitué dans R₁ à R₄ et R₈ sont chacun indépendamment au moins un choisi dans le groupe constitué de: deutérium, un halogène, un cyano, un carboxyle, un nitro, un hydroxyle, un (C1-C30)alkyle, un halo(C1-C30)alkyle, un (C2-C30)alcényle, un (C2-C30)alcynyle, un (C1-C30)alcoxy, un (C1-C30)alkylthio, un (C3-C30)cycloalkyle, un (C3-C30)cycloalcényle, un hétérocycloalkyle de 3 à 7 membres, un (C6-C30)aryloxy, un (C6-C30)arylthio, un hétéroaryle de 5 à 30 membres non substitué ou substitué avec un (C6-C30)aryle, un (C6-C30)aryle non substitué ou substitué avec un hétéroaryle de 5 à 30 membres, un tri(C1-C30)alkylsilyle, un tri(C6-C30)arylsilyle, un di(C1-C30)alkyl(C6-C30)arylsilyle, un (C1-C30)alkyldi(C6-C30)arylsilyle, un amino, un mono- ou di-(C1-C30)alkylamino, un mono- ou di-(C6-C30)arylamino, un (C1-C30)alkyl(C6-C30)arylamino, un (C1-C30)alkylcaibonyle, un (C1-C30)alcoxycarbonyle, un (C6-C30)arylcarbonyle, un di(C6-C30)arylboronyle, un di(C1-C30)alkylboronyle, un (C1-C30)alkyl(C6-C30)arylboronyle, un (C6-C30)aryl(Cl-C30)alkyle et un (C1-C30)alkyl(C6-C30)aryle.

5. Matériau hôte phosphoré selon la revendication 1, dans lequel
R₁ à R₄ et R₈ représentent chacun indépendamment de l'hydrogène, un (C1-C6)alkyle substitué ou non substitué ou un (C6-C15)aryle substitué ou non substitué; ou sont liés à un (ou plusieurs) substituant(s) adjacent(s) pour former un cycle (C3-C12) aromatique, mono- ou polycyclique, substitué ou non substitué.

6. Matériau hôte phosphoré selon la revendication 1, dans lequel
R₃ à R₄ représentent chacun indépendamment un (C1-C6)alkyle non substitué ou un (C6-C15)aryle non substitué ou substitué avec un (C1-C6)alkyle et
R₇ et R₈ représentent chacun indépendamment de l'hydrogène.

7. Matériau hôte phosphoré selon la revendication 1, dans lequel le composé représenté par la formule 1 est choisi dans le groupe constitué de:

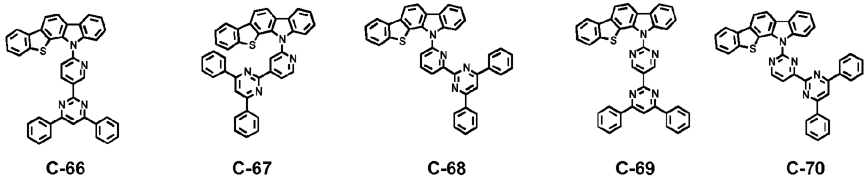

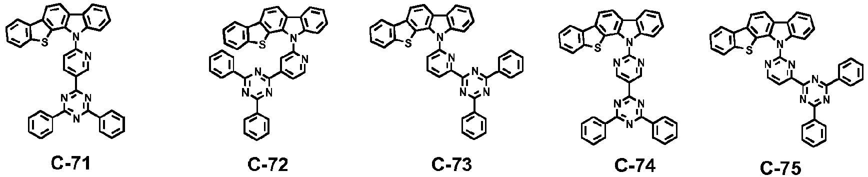

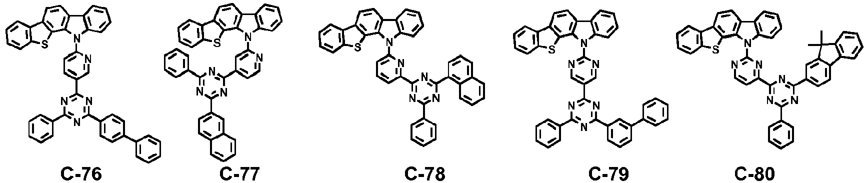

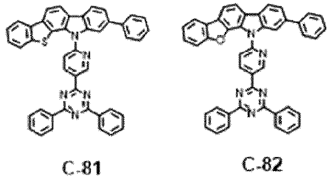

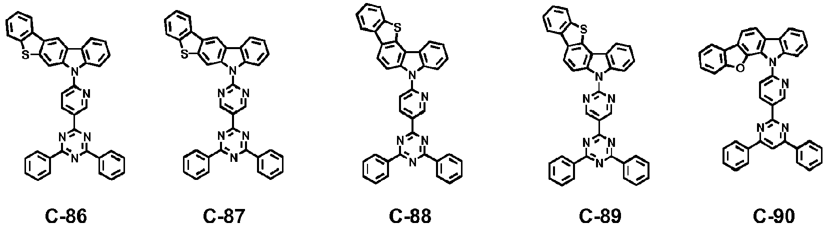

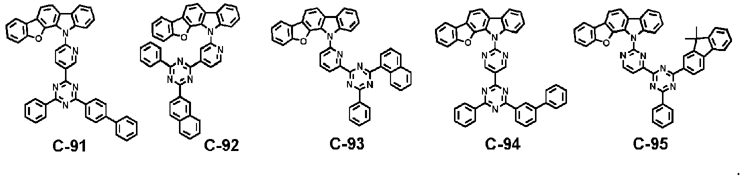

8. Dispositif électroluminescent organique comprenant le matériau hôte phosphoré selon la revendication 1.
